Europäisches Patentamt

European Patent Office

Office européen des brevets

(11) Publication number: **0 477 833 A1**

# EUROPEAN PATENT APPLICATION

(21) Application number: **91116188.3**

(22) Date of filing: **24.09.91**

(51) Int. Cl.⁵: **A61K 37/12**, A61K 7/48

(30) Priority: **28.09.90 IT 2160690**

(43) Date of publication of application:
**01.04.92 Bulletin 92/14**

(84) Designated Contracting States:
**AT BE CH DE DK ES FR GB LI NL**

(71) Applicant: **Sternberg Ruiu, Rosa, Dr.**
**Via Palmanova, 91**
**I-20132 Milano(IT)**

(72) Inventor: **Sternberg Ruiu, Rosa, Dr.**
**Via Palmanova, 91**
**I-20132 Milano(IT)**

(74) Representative: **Dragotti, Gianfranco et al**
**SAIC BREVETTI s.r.l. Viale Bianca Maria, 15**
**I-20122 Milano(IT)**

(54) **Composition for therapeutic and/or cosmetic use for treatment of circulatory diseases and for aesthetic medicine treatments.**

(57) The combination in a composition for topical use of hydrogenated lecithins, hyaluronic acid and elastin, and possibly diachysis factor, is useful in the treatment of pathologies such as varicose veins, phlebitis, oedemas and obstructed veins as well as in applications of aesthetical medicine.

EP 0 477 833 A1

The present invention relates to a composition for therapeutical and/or cosmetic use. More particularly the present invention relates to a composition useful for:

a) the treatment of varicose veins, phlebitis, oedemas, sores induced from varicose veins, veins obstructed owing to sclerosating therapy and more generally for the restoration of the blood circulation and for the normalization of the walls of the blood vessels;

b) the treatments of aesthetic medicine, particularly for the treatment of "couperose" (teleangiectasy), acne, capillaritis of the outer surface of the nose, the limbs and the face, cellulitis and states of tissue relaxation.

It is well known that for several of the above listed diseases the solutions proposed to date for the therapeutical treatment were not effective and often contemplate the recourse to radical ways, namely the surgical intervention: a classical example is that of the varicose veins and of the phlebitis the recovery of which is in most cases obtained by removing the saphena vein.

On the contrary in the case of the above mentioned unaesthetisms, apart from empirical approaches which often borders on quackery, all the treatments adopted to date did not led to certainly positive results, even when were based on products of natural origin. In most cases, as a matter of fact, it was seen that these products and treatments had an utility limited to part of the stated symptoms.

An example is that of the cellulitis for the resolution of which, apart from the recourse to treatments of mechanical nature, one remedy to date adopted on widespread basis has been that of massages for extended time periods using products sometimes of natural origin capable of inducing the reabsorption of the liquids and lipids to which such an unaesthetism can be attributed; in extreme cases a surgical intervention by liposuction is effected.

Another example is that of the tissue relaxation, especially of the face, of the hips and of the thighs.

To date the only treatment capable of truly eliminate such an unaesthetism has been the surgical one, namely the well known "lifting": this treatment, however, has not negligible side effects, mainly that of modifying the face expression.

It has been now found and is the object of the present invention that by combining some products of natural origin, in particular proportions, it is possible to succesfully treat both the pathologies and the unaesthetisms as above listed, by topical applications and in a limited number of times and thus in a reduced treatment time.

The composition according to the present invention is characterized by comprising the following active ingredients:
hydrogenated lecithins, hyaluronic acid, elastin and possibly diachysis factor. More specifically the composition according to the present invention contemplates the following formulation:

| | | |
|---|---|---|
| hydrogenated lecithins weight | 0.7 – 1.2 | percent by |
| hyaluronic acid | 0.47 – 1.2 | " " " |
| elastin | 0.47 – 1.2 | " " " |
| diachysis factor | 60.00 –.70.00 | " " ". |

As already mentioned the diachysis factor is an ingredient of the preferred composition, but is not essential since from the experiments it has been found that the three firstly mentioned components are those responsible of the activity of the composition according to the invention.

Of course besides these active ingredients the conventional vehicles and excipients, per se well known in the preparation of fluid or liquid compositions for topical uses, are contemplated.

Turning now to the single active ingredients:

1) The hydrogenated lecithins or liposomes have been studied and developed as a substitute for the natural lecithins in order to eliminate their drawbacks (bad odour and colour, poor stability and redy oxidability). These substances have found use in the cosmetic field, mostly as thickening agents and carriers for the penetration of other active substances, either of cosmetic or of therapeutical type.

2) The hyaluronic acid is a glycosaminoglycane deprived of sulfur having high molecular weight and present in all tissues, which does interact with a number of biological macromolecules. The use thereof in the cosmetic field is mainly connected to its hydroretention capacity.

3) The elastin is a component of several tissues and organs of the higher animals and of the human beings and is responsible of the extensibility and of the reversible deformability which characterizes all the elastic tissues.The preparation thereof takes place by means of a controlled and mild, enzymatic hydrolysis of bovine tendons, previously defatted and depurated from contaminating compounds of collagenic nature.In the cosmetic field the use thereof is like that of collagen.

4) The diachysis factor or penetration factor is the non chemical name by which the products resulting from the enzymatic and acid hydrolysis of acid mucopolysaccharides deprived of sulfur are indicated. To this factor a number of activities is attributed both of strictly cosmetic nature and of more properly therapeutic nature, these activities being constantly connected to their property of binding the water in the cellular interstices forming a viscous gel acting as shock absorber, lubricant and barrier against the foreign substances.Otherwise stated in this case too a definitely hydroretentive action occurs.

Of course more detailed information on the above cited substabces can be found in the technical literature which in this connection is very abundant.

Form the above short notes it is evident that from the knowledge of the characteristics and properties of the above stated active substances of the composition according to the present invention it was not possible to foresee the therapeutical and cosmetic activities which the same composition has shown through experimental tests on voluntary patients. Turning now to the therapeutical use there is firstly examined the action of the composition of the present invention in the treatment of phlebitis and varicose veins. As it is well known, owing to a pathological alteration of the walls of the blood vessels, the latter are alterated not only as regards the shape, since a dilatation, an elongation and a deviation from the rectilinear pattern occur, but also as regards their permeability. This alteration leads to oedemas occurring owing to substances coming out of the vessels. In the long run these oedemas may originate ulcerations.

The composition of the present invention adopted in the therapy of these cases has the following formulation:

| hydrogenated lecithins | 3.5 g. |
|---|---|
| hyaluronic acid | 2.0 mg. |
| elastin | 2.0 mg. |
| diachysis factor | 294.0 mg. |
| mannitol | 120.0 mg. |
| distilled water | enough |

This composition is applied to the legs, topically, by a daily application and after 4 to 5 days from the beginning of the treatment a diminution of the oedema and of the pain is observed, sidewise accompanied by an increase of the diuresis.

Depending of the seriousness of the pathological situation the treatment is to be repeated by a number of cycles having an average duration of 10 to 15 applications, according to the gravity of the symptoms, and at the end of the treatment a reduction of the symptoms by at least 90% takes place.

The treatment is moreover extended in the presence of other worsening factors such as the smoke, the use of anticonceptional products, the body overweight etc.

Turning now to consider the cosmetic uses of the compositions according to the present invention it is to be noted that:

a) Couperose.

The cyclical treatment with the composition of the present invention causes a progressive reduction of the enlargement and of the reddening of the vessels which are seen in this unaesthetism until these symptoms totally or almost totally disappear.

b) Cellulitis.

In this case the topical application of the composition according to the invention, periodically repeated by cycles of about 15 treatments, leads to a reduction of the unaesthetism by about 90%.

c) Hypertrophic and cyanotic nose.

By applying the composition of the invention a reduction of the unaesthetism is observed, it being

EP 0 477 833 A1

improved by 90%.

d) Lifting.

By applying the composition of the present invention a shrinkage of the skin is observed, mainly localized at the neck , forepart of the ears and eyelids.

The improvement is of the order of 90% and is permanent. Like results are obtained by treating the inner part of the thighs, the abdomen, the breast and the internal part of the arms.

e) Acne.

After a preventive abrasive cleaning of the skin, each pustule is treated with the composition of the invention giving place to a reconstruction and a normalization of the tissue, the improvement being of the order of 90%.

As regards the conditions of topical application of the compositions according to the present invention, there is no need of an extended massage, provided that the liquid or fluid penetrates, the action continuing afterwards without any mechanical or manual intervention for a period of the order of 12 hours after the application.

**Claims**

1. Composition for therapeutic and/or cosmetic use, characterized in that it includes in combination as active ingredients hydroge ated lecithins, hyaluronic acid as well as elastin, together with the usual excipients, vehicles and solubilizing agents for liquid or fluid composition.

2. Composition for therapeutic and/or cosmetic use, according to claim 1, characterized in that it includes in adddition the diachysis factor.

3. Composition for therapeutic and/or cosmetic use, according to claim 1, characterized in the following percentages by weight of said active ingredients:

| | |
|---|---|
| hydrogenated lecithins | 0,71 – 1,2 |
| hyaluronic acid | 0,47 – 1,2 |
| elastin | 0,47 – 1,2 |

4. Composition for therapeutic and/or cosmetic use, according to claim 2, characterized in that said diachysis factor is present in a percentage by weight included between 60 and 70 per cent.

5. Therapeutic and/or cosmetic composition, according to the preceding claims, characterized by the following formulation.

| | |
|---|---|
| hydrogenated lecithins | mg 3,5 |
| hyaluronic acid | mg 2,0 |
| elastin | mg 2,0 |
| diachysis factor | mg 294 |
| mannitol | mg 120 |
| distilled water | enough |

6. Composition, according to the preceding claims, for the therapy of varicose veins, phlebitis, oedema, sores induced from varicose ulcers, obstructed veins owing to a sclerozating therapy as well as for the restoration of circulation and for the normalization of walls of blood vessels.

4

7. Composition, according to the preceding claims, for the treatment of telangiectasia, acne, capillaritis of nose, limbs and face, cellulitis and states of tissue relaxation.

## European Patent Office

# EUROPEAN SEARCH REPORT

Application Number

## EP 91 11 6188

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-2 614 787   (POLA KASEI KOGYO K.K.) <br> * Claims 1-3 * | 1 | A 61 K 37/12 <br> A 61 K 7/48 |
| Y | WO-A-8 707 502   (PHARES PHARMACEUTICAL RE- SEARCH N.V.) <br> * Claims 1,5; abstract * | 1 | |
| Y | DATABASE DERWENT, WPIL, accession no. 87-296405 [42], Derwent Publications Ltd, London, GB; <br> & <br> & JP-A-62 209 010 (KOBAYASHI KOSE K.K.) 14-09-1987 <br> * Abstract * | 1 | |
| A | PATENT ABSTRACTS OF JAPAN, vol. 8, no. 41 (C-211)[1478], 22nd February 1984; <br> & JP-A-58 201 708 (ASAHI KASEI KOGYO K.K.) 24-11-1983 | 1 | |
| A | FR-A-2 356 427   (LABORATOIRES ANA) <br> * Page 2, lines 2-15; claim 1 * | 1,2,4,7 | |
| A | MINERVA CARDIOANGIOLOGICA, vol. 34, no. 4, April 1986, pages 251-254; A. LAPILLI et al.: "Metodiche alternative nel trattamento dell'ulcera varicosa e risultati" <br> * Pages 251,254 * | 1,6 | TECHNICAL FIELDS SEARCHED (Int. Cl.5) <br><br> A 61 K |

The present search report has been drawn up for all claims

| Place of search | Date of completion of search | Examiner |
|---|---|---|
| The Hague | 11 December 91 | SITCH W.D.C. |